# EUROPÄISCHE PATENTANMELDUNG

(11) **EP 4 548 945 A1**
(43) Veröffentlichungstag der Anmeldung: **07.05.2025**
(21) Anmeldenummer: 24187727.3
(22) Anmeldetag: 10.07.2024
(51) Int. Cl.: A61M 1/34, A61M 1/14

(54) **OPTISCHE ERFASSUNGSEINRICHTUNG FÜR EIN FLUID IN EINEM LEITUNGSABSCHNITT EINES MEDIZINISCHEN GERÄTES**

(30) Priorität: 13.07.2023 DE 102023118626
(71) Anmelder: B. Braun Avitum AG, 34212 Melsungen (DE)
(72) Erfinder: BRESSAU, Ernst, 34323 Malsfeld (DE); HOLLSTEIN, Rolf, 36211 Alheim-Heinebach (DE)
(74) Vertreter: Winter, Brandl - Partnerschaft mbB

(57) **Zusammenfassung**

Offenbart sind eine optische Erfassungseinrichtung und eine Anordnung mit einer derartigen optischen Erfassungseinrichtung und mit einem lichtdurchlässigen Leitungsabschnitt (1), in dem ein Fluids (2) geführt ist. Die Erfassungseinrichtung hat genau eine Messlichtquelle (6) und genau einen Messlichtsensor (10), wobei sich von der Messlichtquelle (6) zu dem Messlichtsensor (10) entlang einer Messlichtachse (16) ein Messlichtbereich (18) erstreckt. Für einen Selbsttest bzw. Funktionstest ist benachbart zur Messlichtquelle (6) ein Testlichtsensor (8) angeordnet, der in optische Wirkverbindung mit der Messlichtquelle (6) bringbar ist, und benachbart zum Messlichtsensor (10) ist eine Testlichtquelle (12) angeordnet, die in optische Wirkverbindung mit dem Messlichtsensor (10) bringbar ist.

## Beschreibung

### Technisches Gebiet

Die Offenbarung betrifft den vorzugsweise automatischen Selbsttest bzw. Funktionstest einer optischen Erfassungseinrichtung für ein Fluid in einem Leitungsabschnitt eines medizinischen Gerätes und insbesondere eine entsprechende optische Erfassungseinrichtung gemäß dem Oberbegriff des Anspruchs 1.

### Hintergrund der Offenbarung

Besonderes Augenmerk liegt bei medizinischen Geräten wie z.B. Dialysegeräten auf optischen Sensorverfahren, welche den Inhalt z.B. das Blut des Leitungssystems berührungslos und zerstörungsfrei analysieren können. Berührungslos bedeutet in diesem Zusammenhang, dass das Blut nicht mit einem anderen Material als dem Standardmaterial des Leitungssystems in Berührung kommt. Zerstörungsfrei bedeutet in diesem Zusammenhang, dass die Messmethode keinen negativen Einfluss auf die zellulären oder molekularen Bestandteile des Blutes ausübt, diese schwächt oder gar zerstört. Dies ist z.B. bei der Dialyse besonders wichtig, da bei Dialysepatienten die Bildung von roten Blutkörperchen beeinträchtigt ist und jeder Verlust derselben den Gesundheitszustand und das Wohlbefinden des Patienten dauerhaft beeinträchtigen kann.

Bei Patienten mit eingeschränkter oder fehlender Nierenfunktion werden Abfallprodukte des natürlichen Stoffwechsels durch Nierenersatz- bzw. Dialyseverfahren entfernt. Dabei erfolgt die Entfernung der Stoffe aus dem Blut, welches dem Patienten entnommen und extrakorporal geführt wird, durch den Kontakt des Blutes mit einer Dialysierflüssigkeit, wobei Blut und Dialysierflüssigkeit nicht direkt, sondern über eine extrakorporal angeordnete Membran miteinander in Kontakt stehen. Die Dialysierflüssigkeit ist mit verschiedenen Salzen versetzt und ruft somit diffusive und konvektive Effekte hervor, die für den Stofftransport vom Blut in die Dialysierflüssigkeit über die Membran verantwortlich sind. Nach erfolgter Entfernung eines Teils der Abfallstoffe wird das so behandelte Blut dem Patienten wieder zu geführt.

Bekannte optische Sensoren bei medizinischen Geräten wie z.B. Dialysegeräten sind z.B. Hämatokritsensoren (basierend auf rotem und infrarotem Licht), Blutleckdetektoren (messen eine Rotfärbung in Dialysierflüssigkeit im Dialysierflüssigkeitskreislauf) und Rotsensoren (zeigen die Präsenz von Blut im Blutschlauch an).

Die vorliegende Offenbarung betrifft nunmehr eine optische Erfassungseinrichtung (insbesondere einen Rotsensor) für ein Fluid (insbesondere für Blut) in einem Leitungsabschnitt (insbesondere in einem Schlauchabschnitt) eines medizinischen Gerätes wie z.B. eines Dialysegerätes. Die optische Erfassungseinrichtung ist zur Durchführung eines vorzugsweise automatischen Selbsttests ausgebildet bzw. vorbereitet und ausgelegt.

### Stand der Technik

Die EP 2 767 821 A1 offenbart eine optische Erfassungseinrichtung mit einer Lichtquelle, deren Licht entlang einer Messtrecke durch ein Fluid gerichtet ist, wobei das Licht von einem Lichtdetektor erfasst wird. Zur Durchführung eines Selbsttests ist ein Referenz-Lichtdetektor vorgesehen, der an der gleichen Seite des zu analysierenden Fluids angeordnet ist, wie der Lichtquelle. Dabei ist der Referenz-Lichtdetektor unmittelbar benachbart zur Lichtquelle angeordnet und an diese optisch gekoppelt.

Die EP 2 605 810 B1, die EP 2 579 910 B1 und die EP 2 579 911 B1 offenbaren jeweils eine optische Erfassungseinrichtung mit einem Referenz-Lichtdetektor, der an der gleichen Seite des zu analysierenden Fluids angeordnet ist, wie der Lichtquelle. Dabei ist ein Referenz-Lichtdetektor seitlich zur Messstrecke bzw. Richtung des Lichts beabstandet zur Lichtquelle und zur Messtrecke angeordnet. Der Referenz-Lichtdetektor empfängt Licht von der Lichtquelle, das über einen Lichtteiler abgezweigt wird.

Aus hausinternem Stand der Technik ist weiterhin eine optische Erfassungseinrichtung für Fluid (Blut oder Kochsalzlösung oder Luft) eines Blutschlauchabschnitts eines Dialysegerätes mit zwei redundanten Kanälen bekannt. Jeder Kanal hat an einer ersten Seite des Schlauches eine LED und an einer der ersten Seite gegenüberliegenden zweiten Seite des Blutschlauches eine Fotodiode. Die beiden Messstrecken sind zueinander parallel und rechtwinkelig zum Schlauchabschnitt ausgerichtet. Die beiden Messtrecken sind entlang dem Leitungsabschnitt bzw. der Strömungsrichtung des Fluids zueinander beabstandet. Von den beiden LED's kann ein jeweiliges Sendesignal und ein jeweiliges Testsignal in Form von Licht entlang der jeweiligen Messtrecke durch den Schlauchabschnitt abgestrahlt werden. Damit ist eine Betriebssicherheit gegeben, denn durch einen Abgleich kann erkannt werden, wenn ein Kanal ausfällt.

### Kurzbeschreibung der Offenbarung

Aufgabe der vorliegenden Offenbarung ist es, eine optische Erfassungseinrichtung für ein in einem lichtdurchlässigen Leitungsabschnitt aufgenommenes Fluid (insbesondere für in einem Schlauchabschnitt geführtes Blut) und eine Anordnung mit einer derartigen optischen Erfassungseinrichtung zu schaffen, die zur Durchführung eines Selbsttests bzw. Funktionstests ausgelegt und vorbereitet sowie eingerichtet ist. Dabei sollen der Platzbedarf und der vorrichtungstechnische Aufwand und die nötige Rechenleistung zum Betrieb und beim Selbsttest bzw. Funktionstest verringert werden.

Diese Aufgabe wird gelöst mit einer optischen Erfassungseinrichtung mit der Merkmalskombination des Anspruchs 1 und mit einer Anordnung mit der Merkmalskombination des Anspruchs 15.

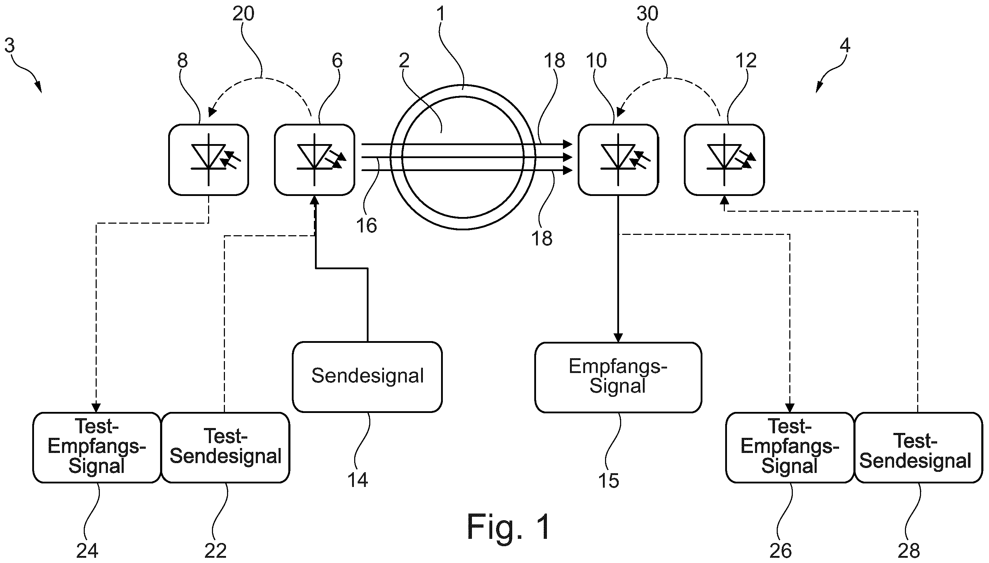

Die optische Erfassungseinrichtung gemäß der Offenbarung hat genau eine Messlichtquelle und genau einen Messlichtsensor, wobei sich von der Messlichtquelle zu dem Messlichtsensor entlang einer Messlichtachse ein Messlichtbereich erstreckt. Die Erfassungseinrichtung ist zur optischen Erfassung eines in einem lichtdurchlässigen Leitungsabschnitt (vorzugsweise Schlauchabschnitt) geführten Fluids eingerichtet und ausgelegt. Unter dem Begriff "Messlichtbereich" ist ein vorzugsweise rotationssymmetrischer länglicher, vorzugsweise sehr schmaler Raum zu verstehen, der von Messlicht definiert bzw. geflutet wird, das von der Messlichtquelle zum Messlichtsensor strahlt. In anderen Worten ausgedrückt ist der "Messlichtbereich" ein Teilraumbereich eines fluidgefüllten Raumes (lichtdurchlässiger Leitungsabschnitt), wobei nur dieser Teilraumbereich vom Messlicht in messtechnisch beabsichtigter Weise durchstrahlt wird. Dieser so definierte Messlichtbereich durchsetzt den fluidgefüllten Raum / Leitungsabschnitt und damit das zu erfassende Fluid. Die Messlichtachse ist vorzugsweise die Mittelachse des Messlichtbereiches und ferner vorzugsweise senkrecht zum fluidgefüllten Raum / Leitungsabschnitt. An einer (radial betrachtet) ersten Seite des Leitungsabschnitts (d.h. im Bereich eines ersten Umfangbereichs des lichtdurchlässigen Leitungsabschnitts, beispielsweise auf 9:00 Uhr gemäß der Fig. 1) ist benachbart zur Messlichtquelle (vorzugsweise auf einer dem Leitungsabschnitt im Wesentlichen abgewandten Seite der Messlichtquelle) ein Testlichtsensor angeordnet, der in optische (lichttechnische) Wirkverbindung mit der Messlichtquelle bringbar ist.

An einer dieser so definierten ersten Seite (radial betrachtet bzw. diametral) gegenüberliegenden zweiten Seite (Umfangsbereich) des Leitungsabschnitts (beispielsweise auf 3:00 gemäß der Fig. 1) ist der Messlichtsensor platziert, wobei benachbart zum Messlichtsensor (vorzugsweise auf einer dem Leitungsabschnitt im Wesentlichen abgewandten Seite des Messlichtsensors) eine Testlichtquelle angeordnet ist, die in optische (lichttechnische) Wirkverbindung mit dem Messlichtsensor bringbar ist. "Wirkverbindung" bedeutet, dass von den genannten Lichtquellen zu den genannten Lichtsensoren Licht für einen Funktionstest und/oder Selbsttest übertragbar ist.

Damit ist eine optische Erfassungseinrichtung geschaffen, die einerseits ihre Messlichtquelle mittels des Testlichtsensors auf der ersten Seite des Leitungsabschnitts testen kann, ohne dass das dazu benötigte Testlicht von der einen auf die andere Seite des Leitungsabschnitts und insbesondere nicht durch den Leitungsabschnitt und das Fluid hindurchgeführt werden muss, und dabei von diesen absorbiert wird. Letzteres kann somit als Einkanal-Prinzip bezeichnet werden, wonach nur ein Messlicht (einkanalig) ausgehend von der Lichtquellenseite zur Messlichtsensorseite geleitet wird, wohingegen das Licht für einen Funktions-/Selbsttest immer auf jener Seite erzeugt und gemessen wird, auf welcher sich die Messlichtquelle bzw. der Messlichtsensor jeweils befinden.

Prinzipiell vergleichbar kann andererseits der Messlichtsensor mittels der Testlichtquelle auf der zweiten Seite des Leitungsabschnitts getestet werden, ohne dass das dazu benötigte Testlicht von der einen auf die andere Seite des Leitungsabschnitts und insbesondere nicht durch den Leitungsabschnitt und das Fluid hindurch- (oder herum-) geführt werden muss und dabei von diesen entweder absorbiert oder mehrfach umgelenkt wird.

Mit der optischen Einkanal-Erfassungseinrichtung gemäß der Offenbarung ergeben sich gegenüber den Erfassungseinrichtungen mit Spiegeln und gegenüber der zweikanaligen Erfassungseinrichtung des Standes der Technik ein verringerter Bauraum und geringere benötigte Rechenleistung für den Selbsttest bzw. Funktionstest. Da entlang dem Leitungsabschnitt beabstandet zueinander statt zwei Messlichtachsen nur eine Messlichtachse nötig ist, ist der Bauraum der Erfassungsvorrichtung entlang dem Leitungsabschnitt in Strömungsrichtung des Fluids verringert und es können Komponenten und Kosten aufgrund des einfacheren Aufbaus eingespart werden. Außerdem wird aufgrund des Einkanal-Prinzips ein eindeutiges Messergebnis erhalten. Bei zweikanaligen Erfassungseinrichtungen mit zwei Messlichtsensoren können im Gegensatz dazu unterschiedliche Messwerte erhalten werden, sodass das Messergebnis nicht eindeutig ist. Somit vereinfacht die optische Einkanal-Erfassungseinrichtung gemäß der Offenbarung die Signalauswertung. Ferner wird eine einfachere Abschottung gegen Fremdlicht aufgrund des vereinfachten Aufbaus ermöglicht.

In anderen Worten ist der Testlichtsensor eingerichtet und/oder ausgelegt und/oder vorgesehen, die Messlichtquelle (auf deren Funktionalität) zu testen/ zu überprüfen bzw. mit ihm die (Funktionalität der) Messlichtquelle zu testen/ zu überprüfen. In noch anderen Worten ist der Testlichtsensor eingerichtet und/oder ausgelegt und/oder vorgesehen, einen Funktionstest und/oder Selbsttest der Messlichtquelle durchzuführen bzw. mit ihm einen Funktionstest und/oder Selbsttest der Messlichtquelle durchzuführen.

In anderen Worten ist die Testlichtquelle eingerichtet und/oder ausgelegt und/oder vorgesehen, den Messlichtsensor (auf dessen Funktionalität) zu testen/ zu überprüfen bzw. mit ihr die (Funktionalität des) Messlichtsensors zu testen/ zu überprüfen. In noch anderen Worten ist die Testlichtquelle eingerichtet und/oder ausgelegt und/oder vorgesehen, einen Funktionstest und/oder Selbsttest des Messlichtsensors durchzuführen bzw. mit ihr einen Funktionstest und/oder Selbsttest des Messlichtsensors durchzuführen.

Da der Funktionstest der Messlichtquelle komplett auf der ersten Seite des Leitungsabschnitts erfolgen kann, ohne dass dazu Testlicht durch den Leitungsabschnitt oder über einen semipermeablen Spiegel um den Leitungsabschnitt herumgeführt werden muss, kann der Testlichtsensor eine geringere Empfindlichkeit als der Messlichtsensor haben. Damit ist die Erfassungseinrichtung vorrichtungstechnisch vereinfacht und kostengünstig.

Da der Test des Messlichtsensors komplett auf der zweiten Seite des Leitungsabschnitts erfolgen kann, ohne dass dazu Testlicht durch den Leitungsabschnitt strahlen muss und dabei von diesem absorbiert wird, kann die Testlichtquelle eine geringere Lichtstärke als die Messlichtquelle haben. Damit ist die Erfassungseinrichtung vorrichtungstechnisch vereinfacht und kostengünstig.

Die beiden Lichtquellen sind vorzugsweise LED's und/oder die beiden Lichtsensoren sind vorzugsweise Fotodioden oder Fototransistoren.

Z.B. bei Verwendung einer handelsüblichen LED als Messlichtquelle ist der auf den Messlichtsensor auftreffende Messlichtbereich oftmals nur ein (z.B. konzentrischer) Teilbereich eines Hauptlichtbereichs, der von der Messlichtquelle definiert ist bzw. abgestrahlt wird. Bei einer bevorzugten Ausgestaltung der Erfassungseinrichtung ist der Testlichtsensor außerhalb des Messlichtbereichs und zumindest abschnittsweise innerhalb des Hauptlichtbereichs angeordnet. Dann kann beim Selbsttest bzw. Funktionstest das vergleichsweise intensive Hauptlicht der Messlichtquelle von Testlichtsensor erfasst werden, ohne dass dieses durch den Leitungsabschnitt strahlen oder über einen semipermeablen Spiegel geführt muss.

Z.B. bei Verwendung einer handelsüblichen LED als Messlichtquelle kann von dieser ein Streulichtbereich definiert sein bzw. abgestrahlt werden, in dem der Messlichtbereich und gegebenenfalls auch der Hauptlichtbereich angeordnet ist/sind. Bei einem anderen bevorzugten Ausführungsbeispiel der Erfassungseinrichtung ist der Testlichtsensor außerhalb des Messlichtbereichs und innerhalb des Streulichtbereichs angeordnet. Dann kann beim Selbsttest das vergleichsweise schwache Streulicht der Messlichtquelle von Testlichtsensor erfasst werden. Damit ergeben sich Vorteile bei der platzsparenden Anordnung und bei der Montage der Messlichtquelle und des Testlichtsensors an der ersten Seite des Leitungsabschnitts.

Wenn eine Mittelachse des Testlichtsensors rechtwinklig zur Messlichtachse angeordnet ist, ergeben sich Vorteile bei der platzsparenden Anordnung und bei der Montage der Messlichtquelle und des Testlichtsensors an der ersten Seite des Leitungsabschnitts.

Vorzugsweis ist an der ersten Seite des Leitungsabschnitts eine erste Leiterplatte vorgesehen, an der die Messlichtquelle und der Testlichtsensor befestigt sind.

Bei einer besonders bevorzugten Ausgestaltung der Erfassungseinrichtung ist die Testlichtquelle an der zweiten Seite außerhalb des Messlichtbereichs angeordnet, damit die Testlichtquelle keine Abschattung des auf den Messlichtsensor auftreffenden Messlichts bewirkt.

Bei einem bevorzugten Ausführungsbeispiel der Erfassungseinrichtung ist der Messlichtsensor in einem Streulichtbereich der Testlichtquelle angeordnet. Dann kann beim Selbsttest bzw. Funktionstest das (vergleichsweise schwache) Streulicht der Testlichtquelle von Messlichtsensor erfasst werden. Damit ergeben sich Vorteile bei der platzsparenden Anordnung und bei der Montage der Testlichtquelle und des Messlichtsensors an der zweiten Seite des Leitungsabschnitts.

Wenn eine Abstrahlrichtung der Testlichtquelle rechtwinklig zur Messlichtachse angeordnet ist, ergeben sich Vorteile bei der platzsparenden Anordnung und bei der Montage der Testlichtquelle und des Messlichtsensors an der zweiten Seite des Leitungsabschnitts.

Vorzugsweis ist an der zweiten Seite des Leitungsabschnitts eine zweite Leiterplatte vorgesehen, an der der Messlichtsensor und die Testlichtquelle befestigt sind.

Bei einer bevorzugten Ausführungsform der Erfassungseinrichtung sind die beiden Leiterplatten parallel zueinander und senkrecht zur Messlichtachse angeordnet.

Die Messlichtquelle oder der Messlichtsensor kann ein quaderförmiges Gehäuse oder einen quaderförmigen Körper haben, das/der mit (s)einer Großseite oder mit (s)einem Boden an der Leiterplatte anliegt oder angelötet ist. Vorzugsweise haben die Messlichtquelle und der Messlichtsensor jeweils ein quaderförmiges Gehäuse oder einen quaderförmigen Körper, das/der mit (s)einer jeweiligen Großseite bzw. mit (s)einem Boden an der jeweiligen Leiterplatte anliegt bzw. angelötet ist. Damit sind im Falle von flach ausgestalteten LED-Bausteinen (z.B. SMD-Bauteilen) diese flach auf der jeweiligen Leiterplatte montiert.

Die Testlichtquelle oder der Testlichtsensor kann ein quaderförmiges Gehäuse oder einen quaderförmigen Körper haben, das/der mit (s)einer Schmalseite an der Leiterplatte anliegt. Vorzugsweise haben die Testlichtquelle und der Testlichtsensor jeweils ein quaderförmiges Gehäuse oder einen quaderförmigen Körper, das/der mit (s)einer jeweiligen Schmalseite an der jeweiligen Leiterplatte anliegt. Damit sind im Falle von flach ausgestalteten LED-Bausteinen (z.B. SMD-Bauteilen) diese hochkant oder senkrecht auf der jeweiligen Leiterplatte montiert.

Wenn die Messlichtquelle und/oder die Testlichtquelle grünes Licht mit einer Wellenlänge von 490 bis 575 nm, das heißt zwischen 490 und 575 nm, vorzugsweise 520 bis 530 nm abstrahlen, kann besonders gut erkannt werden, ob es sich bei dem in dem Leitungsabschnitt aufgenommenen Fluid um ein rotes oder farbloses Fluid handelt. Insbesondere kann erkannt werden, ob sich in dem Leitungsabschnitt Blut oder ein anderes Fluid (Kochsalzlösung oder Luft) befindet.

Die Anordnung gemäß der Offenbarung weist eine vorbeschriebene optische Erfassungseinrichtung und einen lichtdurchlässigen Leitungsabschnitt auf, durch den sich der Messlichtbereich und die Messlichtachse erstrecken. An einander gegenüberliegenden Seiten des Leitungsabschnitts sind einerseits die Messlichtquelle zusammen mit dem Testlichtsensor und andererseits der Messlichtsensor zusammen mit der Testlichtquelle angeordnet.

Bei einer besonders bevorzugten Ausgestaltung der Anordnung ist der Leitungsabschnitt ein Blutschlauchabschnitt, und der Messlichtsensor und der Testlichtsensor sind Rotsensoren. Damit kann die Anordnung an einem Blutschlauch eines Dialysegeräts eingebaut und verwendet werden, z.B. an dem Blutschlauch, der zurück zum Patienten geführt wird.

### Kurzbeschreibung der Figuren

Nachstehend wird ein bevorzugtes Ausführungsbeispiel der vorliegenden Offenbarung auf der Basis der zugehörigen Figuren beschrieben.
Figur 1 ist ein Funktionsschema des Ausführungsbeispiels der Anordnung mit Erfassungsvorrichtung der vorliegenden Offenbarung;
Figur 2 ist eine geschnittene Darstellung des Ausführungsbeispiels aus Figur 1; und
Figur 3 ist eine perspektivische Darstellung von Bauteilen der ersten Seite des Ausführungsbeispiels der Erfassungsvorrichtung aus den Figuren 1 und 2.

### Figurenbeschreibung

Figur 1 ist ein Funktionsschema des Ausführungsbeispiels der Anordnung mit Erfassungsvorrichtung gemäß der vorliegenden Offenbarung. Die Erfassungsvorrichtung hat einen (lichtdurchlässiger) Leitungsabschnitt 1, der in Fig. 1 im Querschnitt dargestellt ist und der beim gezeigten Ausführungsbeispiel beispielsweise von einem Blutschlauchabschnitt gebildet sein kann, welcher zwischen einer extrakorporalen Blutbehandlungsmaschine wie z.B. ein Dialysegerät und einem Patienten angeordnet ist und in dem Blut nach erfolgter Entfernung von Abfallstoffen zum Patienten zurückgeführt wird. In anderen Worten ausgedrückt ist eine extrakorporale Blutbehandlungsmaschine, bzw. eine Dialysemaschine vorgesehen, mit einem extrakorporalen Blutkreislauf vorzugsweise aufweisend einen (Patienten-) Blutentnahme-Leitungsabschnitt (-schlauch), einen (Patienten-) Blutrückführ-Leitungsabschnitt (-schlauch) und ggf. einen diese beiden Abschnitte verbindenden Dialysefilter, wobei am/im Blutentnahme-Leitungsabschnitt und/oder Blutrückführ-Leitungsabschnitt der lichtdurchlässige Leitungsabschnitt 1 der Erfassungsvorrichtung angeordnet/integriert ist.

Der lichtdurchlässige Leitungsabschnitt 1 ist vorzugsweise aus Silikon oder PVC gefertigt, kann aber auch als Glas- oder Kunststoff-Glasröhrchen ausgebildet sein. In dem lichtdurchlässigen Leitungsabschnitt als Teil des nicht weiter dargestellten Blutschlauchabschnitts 1 soll mittels der (Lichtwellen verwendenden) Erfassungsvorrichtung ermittelt bzw. erkannt werden, ob sich in dem lichtdurchlässigen Leitungsabschnitt des Blutschlauchabschnitts Blut des Patienten oder eine andere (Spül-) Flüssigkeit wie z.B. Kochsalzlösung oder (lediglich) Luft befindet.

Radial zum Blutschlauchanschnitt 1 betrachtet an (vorzugsweise diametral) gegenüberliegenden Seiten ist einerseits eine erste Seite (Umfangsbereich) 3 und andererseits eine zweite Seite (Umfangsbereich) 4 des lichtdurchlässigen Leitungsabschnitts definiert. An der ersten Seite 3 sind eine (Haupt-) Messlichtquelle 6 und ein Testlichtsensor 8 angeordnet. An der zweiten Seite 4 sind ein Messlichtsensor 10 und eine (Neben-)Testlichtquelle 12 angeordnet.

Im Normalbetrieb der so gebildeten optischen Erfassungsvorrichtung für das in dem lichtdurchlässigen Leitungsabschnitt 1 aktuell befindlichen Fluid 2 empfängt die Messlichtquelle 6 ein Sendesignal 14 von einer nicht weiter gezeigten Steuer-/Regelungseinrichtung, welches die Messlichtquelle 6 dazu ansteuert, dass sie entlang einer quer durch den Blutschlauch 1 gerichteten Messlichtachse 16 ein Mess-Licht abgibt, welches den lichtdurchlässigen Leitungsabschnitt 1 durchdringt und das (abzüglich bestimmter Absorptionsanteile aufgrund des im Leitungsabschnitt enthaltenen Fluids) von dem Messlichtsensor 10 an der anderen, zweiten Seite 4 empfangen wird, so dass dieser ein entsprechendes Empfangssignal 15 generiert und an die nicht weiter gezeigte Steuer-/Regelungseinrichtung abgibt.

Das von der Messlichtquelle 6 insgesamt abgegebene Licht lässt sich hierbei prinzipiell in drei Teile oder räumlich Lichtbereiche gliedern:
- Es wird ein Hauptlichtbereich des von der Messlichtquelle 6 abgegebenen Lichts definiert, der anschaulich ausgedrückt der Hauptlichtkegel der Messlichtquelle 6 sein kann, wobei das Hauptlicht vergleichsweise hohe Lichtstärke hat.
- Da von diesem Hauptlicht eventuell nur ein Teil präzise auf den auf der zweiten Seite 4 angeordneten Messlichtsensor 10 ausgerichtet (gebündelt) ist, wird dieser Teil Messlichtbereich 18 genannt. Dieser ist vorzugsweise kleiner als der Durchmesser des lichtdurchlässigen Leitungsabschnitts 1. Der Messlichtbereich 18 ist also ein vorzugsweise konzentrischer Teilbereich des Hauptlichtbereichs (und auch des Querschnitts des lichtdurchlässigen Leitungsabschnitts 1) und ist dadurch definiert, dass dessen (gesamtes) Messlicht auf den Messlichtsensor 10 ausgerichtet (gebündelt) ist. In Figur 1 sind die umfänglichen Grenzen des Messlichtbereiches 18 in Form von äußersten Messlichtstrahlen (obere und untere Pfeile) schematisch dargestellt. In Sonderfällen, in denen das gesamte von der Messlichtquelle 6 abgegebene Hauptlicht auf den Messlichtsensor 10 (präzise) ausgerichtet (gebündelt) ist, entspricht der Messlichtbereich 18 dem Hauptlichtbereich.
- Weiterhin ist der Hauptlichtbereich noch von einem (nicht auf den Messlichtsensor 10 ausgerichteten) Streulichtbereich 20 umgeben, der gegenüber dem Hauptlichtbereich ggf. zwar die gleiche Lichtfrequenz jedoch sicherlich eine verminderte Lichtstärke hat. Von diesem Streulichtbereich 20 ist in Figur 1 symbolisch nur ein Streulichtstrahl (gebogener Pfeil) dargestellt.

Die so definierten drei (oder in Sonderfällen zwei) Lichtbereiche sind Räume, die in einem Normalbetrieb der optischen Erfassungsvorrichtung mit Licht geflutet werden.

In einem automatischen Testbetrieb bzw. bei einem automatischen Funktionstest der optischen Erfassungsvorrichtung wird einerseits an der ersten Seite 3 die Funktion der Messlichtquelle 6 getestet, in dem an sie ein (vorbestimmtes) elektrisches Testsendesignal 22 (zur Erzeugung eines Lichtfrequenz- und/oder Lichtstärkevorbestimmten Lichts) übermittelt wird, wobei/wodurch (gleichzeitig) von dem Testlichtsensor 8 ein elektrisches Testempfangssignal 24 erzeugt wird. Das Testempfangssignal 24 hängt beim, in den Figuren gezeigten Ausführungsbeispiel vom empfangenen Streulicht 20 ab, das der Testlichtsensor 8 von der Messlichtquelle 6 empfängt.

Bei einem nicht gezeigten alternativen Ausführungsbeispiel kann der Testlichtsensor 8 ein elektrisches Testempfangssignal erzeugen, das abweichend von dem zuvor beschriebenen Ausführungsbeispiel vom Hauptlicht abhängt, welches der Testlichtsensor 8 von der Messlichtquelle 6 empfängt. Bei diesem nicht gezeigten alternativen Ausführungsbeispiel ist der Testlichtsensor 8 folglich im Hauptlichtbereich aber nicht im Messlichtbereich der Messlichtquelle 6 angeordnet.

In dem automatischen Testbetrieb der optischen Erfassungsvorrichtung des in Figur 1 gezeigten Ausführungsbeispiels wird weiterhin an der zweiten Seite 4 die Funktion des Messlichtsensors 10 getestet, in dem an die an der zweiten Seite 4 angeordneten Testlichtquelle 12 ein elektrisches Testsendesignal 28 zur Erzeugung eines vorbestimmten Testlichts übermittelt wird, wobei (gleichzeitig) von dem Messlichtsensor 10 ein elektrisches Testempfangssignal 26 erzeugt wird, das bevorzugt von einem Streulicht 30 (des Testlichts) abhängt, das der Messlichtsensor 10 von der Testlichtquelle 12 empfängt.

Bei einem nicht gezeigten alternativen Ausführungsbeispiel kann der Messlichtsensor 10 ein elektrisches Testempfangssignal in Abhängigkeit des von der Testlichtquelle 12 empfangenen Hauptlicht des Testlichts erzeugen. Dazu wird die Testlichtquelle 12 derart an der zweiten Seite angeordnet, dass ihr Hauptlicht direkt auf den Messlichtsensor 10 strahlt.

Grundsätzlich wird also ausschließlich nur das Hauptlicht der Messlichtzelle 6 ausgehend von der ersten Seite 3 in Richtung hin zur zweiten Seite 4 geleitet und nicht, wie aus dem Stand der Technik bekannt auch ein Testlicht (entweder durch den lichtdurchlässigen Leitungsabschnitt oder um diesen zumindest teilweise herum), sodass die Erfassungsvorrichtung prinzipiell als auch als (sicherer) Einkanal-(Rot-) Lichtsensor bezeichnet werden kann.

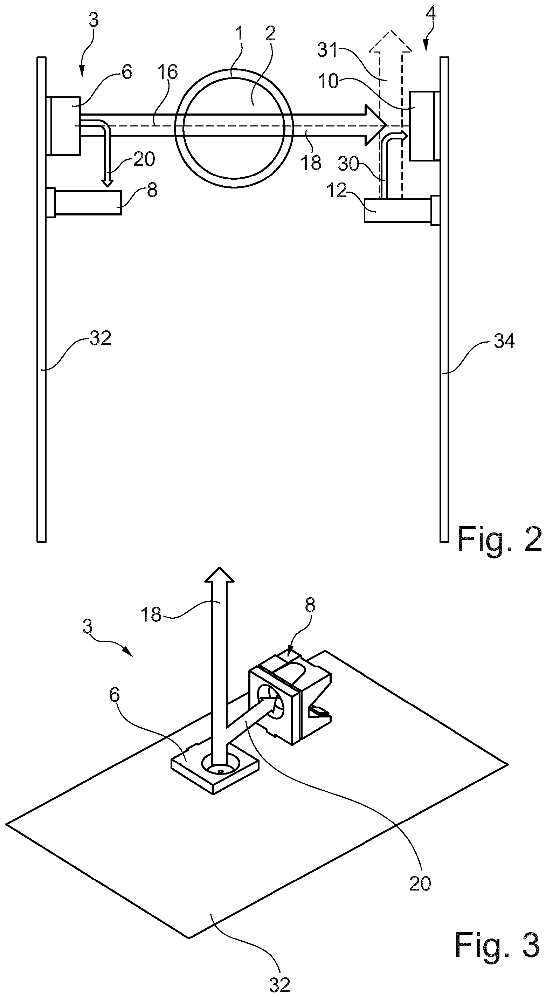

Figur 2 ist eine geschnittene Darstellung der Anordnung aus Figur 1 bestehend aus der (optischen) Erfassungsvorrichtung, deren Lichtquellen und Sensoren an den beiden Seiten 3, 4 des lichtdurchlässigen Leitungsabschnitts 1 des Blutschlauchabschnitts angeordnet ist. An der ersten Seite 3 ist senkrecht zur Messlichtachse 16 eine erste Leiterplatte 32 angeordnet, und an der zweiten Seite 4 ist senkrecht zur Messlichtachse 16 eine zweite Leiterplatte 34 angeordnet. Damit sind die beiden Leiterplatten 32, 34 parallel zueinander ausgerichtet.

Beim gezeigten Ausführungsbeispiel sind die beiden Lichtquellen 6, 12 LED-Bausteine und die beiden Lichtsensoren 8, 10 sind Fotodioden oder Fototransistoren. Die LED-Bausteine und die Fotodioden oder Fototransistoren sind oberflächenmontierte Bauteile (SMD-Bauteile) mit quaderförmigen Gehäusen oder Körpern.

Es ist dargestellt, dass die Messlichtquelle 6 und die Messlichtsensor 10 mit ihren Großseiten bzw. Böden (quasi liegend) an der jeweiligen Leiterplatte 32, 34 angelötet sind, während der Testlichtsensor 8 und die Testlichtquelle 10 mit ihren Schmalseiten bzw. Rändern (quasi hochkant stehend) an der jeweiligen Leiterplatte 32, 34 angelötet sind.

Eine (nicht gezeigte) Mittelachse des Testlichtsensors 8 und eine Abstrahlrichtung 31 der Testlichtquelle 12 schneidet die Messlichtachse 16 rechtwinklig.

Durch die weitgehende 90°-Architektur der Erfassungsvorrichtung ergibt sich, dass der automatische Selbsttest bzw. Funktionstest jeweils mit Streulicht 20, 30 durchgeführt wird. Außerdem ergibt sich daraus eine platzsparende Anordnung der Baugruppen in unmittelbarer Nähe zu dem Außenmantel des Blutschlauchabschnitts 1, wobei entlang dem Blutschlauchabschnitts 1 in Strömungsrichtung des Bluts betrachtet minimaler Bauraum benötigt wird.

Figur 3 ist eine perspektivische Darstellung von Bauteilen der ersten Seite 3 des Ausführungsbeispiels der Erfassungsvorrichtung aus den Figuren 1 und 2. Es sind die erste Leiterplatte 32 und die Messlichtquelle 6 und der Testlichtsensor 8 gezeigt.

Die Messlichtquelle 6 strahlt Messlicht entlang des Messlichtbereichs 18 in Richtung hin zum Leitungsabschnitt 1 (vgl. Figuren 1 und 2). Dabei entsteht Streulicht 20, das von dem Testlichtsensor 8 unmittelbar erfasst werden kann, also ohne dass das Licht über einen Spiegel gemäß dem Stand der Technik ab-/umgelenkt werden muss, und ohne dass das Testlicht den lichtdurchlässigen Leitungsabschnitt 1 und das darin enthaltene Fluid durchdringen muss, wie es bei der zweikanaligen Erfassungsvorrichtung des Standes der Technik nötig ist.

Abweichend vom gezeigten Ausführungsbeispiel sind z.B. auch nicht parallele Anordnungen der beiden Leiterplatten möglich. So können die beiden Leiterplatten auch einen rechten Winkel zueinander aufweisen. Dann liegt entweder die Messlichtquelle mit ihrer Großseite und/oder mit ihrem Boden an der ersten Leiterplatte an, während der Messlichtsensor mit seiner Schmalseite an der zweiten Leiterplatte anliegt. Oder es liegt umgekehrt die Messlichtquelle mit ihrer Schmalseite an der ersten Leiterplatte an, während der Messlichtsensor mit seiner Großseite und/oder mit seinem Boden an der Leiterplatte anliegt.

### Bezugszeichenliste:

- 1: Leitungsabschnitt / Blutschlauchabschnitt
- 2: Fluid
- 3: erste Seite
- 4: zweite Seite
- 6: Messlichtquelle
- 8: Testlichtsensor
- 10: Messlichtsensor
- 12: Testlichtquelle
- 14: Sendesignal
- 15: Empfangssignal
- 16: Messlichtachse
- 18: Messlicht(bereich)
- 20: Streulicht(bereich) der Messlichtquelle
- 22: Testsendesignal
- 24: Testempfangssignal
- 26: Testempfangssignal
- 28: Testsendesignal
- 30: Streulicht(bereich) der Testlichtquelle
- 31: Abstrahlrichtung der Testlichtquelle
- 32: erste Leiterplatte
- 34: zweite Leiterplatte

## Patentansprüche

1. Optische Erfassungseinrichtung, die zur optischen Erfassung eines in einem lichtdurchlässigen Leitungsabschnitt (1) geführten Fluids (2) eingerichtet und ausgelegt ist, wobei die Erfassungseinrichtung genau eine Messlichtquelle (6) und genau einen Messlichtsensor (10) aufweist, wobei sich von der Messlichtquelle (6) zu dem Messlichtsensor (10) entlang einer Messlichtachse (16) ein Messlichtbereich (18) erstreckt, **dadurch gekennzeichnet, dass** benachbart zur Messlichtquelle (6) ein Testlichtsensor (8) angeordnet ist, der in optische Wirkverbindung mit der Messlichtquelle (6) bringbar ist und der eingerichtet und ausgelegt ist, die Messlichtquelle (6) zu testen, und dass benachbart zum Messlichtsensor (10) eine Testlichtquelle (12) angeordnet ist, die in optische Wirkverbindung mit dem Messlichtsensor (10) bringbar ist und die eingerichtet und ausgelegt ist, den Messlichtsensor (10) zu testen.

2. Optische Erfassungseinrichtung nach Anspruch 1 **dadurch gekennzeichnet, dass** der Testlichtsensor (8) eine geringere Lichtempfindlichkeit als der Messlichtsensor (10) hat.

3. Optische Erfassungseinrichtung nach einem der vorhergehenden Ansprüche **dadurch gekennzeichnet, dass** die Testlichtquelle (12) eine geringere Lichtstärke als die Messlichtquelle (8) hat.

4. Optische Erfassungseinrichtung nach einem der vorhergehenden Ansprüche, wobei von der Messlichtquelle (6) ein Hauptlichtbereich definiert ist, in dem der Messlichtbereich (18) angeordnet ist, **dadurch gekennzeichnet, dass** der Testlichtsensor (8) außerhalb des Messlichtbereichs (18) und zumindest abschnittsweise innerhalb des Hauptlichtbereichs angeordnet ist.

5. Optische Erfassungseinrichtung nach einem der vorhergehenden Ansprüche, wobei von der Messlichtquelle (6) ein Streulichtbereich (20) definiert ist, in dem der Hauptlichtbereich und/oder der Messlichtbereich (18) angeordnet sind/ist, **dadurch gekennzeichnet, dass** der Testlichtsensor (8) außerhalb des Messlichtbereichs und innerhalb des Streulichtbereichs (20) angeordnet ist.

6. Optische Erfassungseinrichtung nach einem der vorhergehenden Ansprüche **dadurch gekennzeichnet, dass** eine Mittelachse des Testlichtsensors (8) rechtwinklig zur Messlichtachse (16) angeordnet ist.

7. Optische Erfassungseinrichtung nach einem der vorhergehenden Ansprüche **gekennzeichnet durch** eine erste Leiterplatte (32), an der die Messlichtquelle (6) und der Testlichtsensor (8) befestigt sind.

8. Optische Erfassungseinrichtung nach einem der vorhergehenden Ansprüche **dadurch gekennzeichnet, dass** der Messlichtsensor (10) in einem Streulichtbereich (30) der Testlichtquelle (12) angeordnet ist.

9. Optische Erfassungseinrichtung nach einem der vorhergehenden Ansprüche **dadurch gekennzeichnet, dass** eine Abstrahlrichtung (31) der Testlichtquelle (12) rechtwinklig zur Messlichtachse (16) angeordnet ist.

10. Optische Erfassungseinrichtung nach einem der vorhergehenden Ansprüche **gekennzeichnet durch** eine zweite Leiterplatte (34), an der der Messlichtsensor (10) und die Testlichtquelle (12) befestigt sind.

11. Optische Erfassungseinrichtung nach den Ansprüchen 7 und 10 **dadurch gekennzeichnet, dass** die beiden Leiterplatten (32, 34) parallel zueinander und senkrecht zur Messlichtachse (16) angeordnet sind.

12. Optische Erfassungseinrichtung nach den Ansprüchen 7 und 10 oder nach Anspruch 11 **dadurch gekennzeichnet, dass** die Messlichtquelle (6) und der Messlichtsensor (10) jeweils ein quaderförmiges Gehäuse oder jeweils einen quaderförmigen Körper haben, das/der mit einer jeweiligen Großseite und/oder mit seinem Boden an der jeweiligen Leiterplatte (32, 34) anliegt.

13. Optische Erfassungseinrichtung nach den Ansprüchen 7 und 10 oder nach Anspruch 11 oder nach Anspruch 12 **dadurch gekennzeichnet, dass** die Testlichtquelle (12) und der Testlichtsensor (8) jeweils ein quaderförmiges Gehäuse oder jeweils einen quaderförmigen Körper haben, das/der mit einer jeweiligen Schmalseite an der jeweiligen Leiterplatte (32, 34) anliegt.

14. Optische Erfassungseinrichtung nach einem der vorhergehenden Ansprüche **dadurch gekennzeichnet, dass** die Messlichtquelle (6) und/oder die Testlichtquelle (12) Licht mit einer Wellenlänge von 490 bis 575 nm, vorzugsweise 520 bis 530 nm abstrahlen.

15. Extrakorporale Blutbehandlungsmaschine aufweisend einen extrakorporalen Blutkreislauf mit einer optischen Erfassungseinrichtung gemäß einem der vorhergehenden Ansprüche und mit einem lichtdurchlässigen Leitungsabschnitt (1), durch den sich die Messlichtachse (16) und der Messlichtbereich (18) erstrecken.
